# FASCICULE DE BREVET EUROPEEN

(11) **EP 3 876 836 B1**
(45) Date de publication et mention de la délivrance du brevet: **29.10.2025**
(21) Numéro de dépôt: 19802108.1
(22) Date de dépôt: 05.11.2019
(51) Int. Cl.: A61B 5/0536, A61B 5/11, A61B 5/107, A61B 5/00

(54) **TEXTILE INTELLIGENT ADAPTE POUR LA DETECTION DE MOUVEMENT ET/OU DE DEFORMATION**
ZUR DETEKTION VON BEWEGUNG UND/ODER VERFORMUNG GEEIGNETES, INTELLIGENTES TEXTIL
SMART TEXTILE SUITABLE FOR DETECTING MOVEMENT AND/OR DEFORMATION

(30) Priorité: 06.11.2018 FR 1860192
(43) Date de publication de la demande: 15.09.2021
(73) Titulaire: INRIA - Institut National de Recherche en Informatique et en Automatique, 78150 Le Chesnay (FR)
(72) Inventeur: PUGACH, Ganna, 33400 Talence (FR); DANEY, David, 33200 Bordeaux (FR)
(74) Mandataire: Vidon Brevets & Stratégie
(86) Numéro de dépôt international: PCT/EP2019/080214
(87) Numéro de publication internationale: WO 2020/094628

(56) Documents cités:
- GANNA PUGACH ET AL: "Neural learning of the topographic tactile sensory information of an artificial skin through a self-organizing map", ADVANCED ROBOTICS, vol. 29, no. 21, 2 November 2015 (2015-11-02), NL, pages 1393 - 1409, XP055617592, ISSN: 0169-1864, DOI: 10.1080/01691864.2015.1092395
- RUSSO S ET AL: "Towards a practical implementation of EIT-based sensors using artificial neural networks", 2017 IEEE SENSORS, IEEE, 29 October 2017 (2017-10-29), pages 1 - 3, XP033281144, DOI: 10.1109/ICSENS.2017.8233910
- RUSSO STEFANIA ET AL: "Towards the Development of an EIT-based Stretchable Sensor for Multi-Touch Industrial Human-Computer Interaction Systems", 19 June 2016, INTERNATIONAL CONFERENCE ON COMPUTER ANALYSIS OF IMAGES AND PATTERNS. CAIP 2017: COMPUTER ANALYSIS OF IMAGES AND PATTERNS; [LECTURE NOTES IN COMPUTER SCIENCE; LECT.NOTES COMPUTER], SPRINGER, BERLIN, HEIDELBERG, PAGE(S) 563 - 573, ISBN: 978-3-642-17318-9, XP047349659
- FRANCESCO VISENTIN ET AL: "The Development of a Flexible Sensor for Continuum Soft-Bodied Robots", 31 December 2017 (2017-12-31), XP055618174, Retrieved from the Internet <URL:https://pdfs.semanticscholar.org/aea9/d141098befd1b4315f64444bec57ea4e3bdd.pdf> [retrieved on 20190904]

## Description

L'invention concerne le domaine des textiles dits intelligents.

Les textiles intelligents sont un sous-ensemble des technologies portables (« *wearable technologies* » en anglais). Les vêtements utilisant ces textiles permettent aux personnes les portant d'interagir avec le vêtement, de mesurer son activité physique, de commander son téléphone à distance, etc.

Cela est réalisé par l'introduction de composants informatiques, numériques ou électroniques, mais également par l'utilisation de matériaux polymères innovants ou des matériaux chromiques, ou encore des fibres et matériaux conducteurs.

La plupart des applications d'un vêtement intelligent se trouvent dans les domaines tels que la santé et le sport. Par exemple, la société BioSerenity propose un vêtement intelligent pour la détection de crises d'épilepsie. La société Cityzen Sciences propose des vêtements intelligents destinés aux sportifs. Grâce à des capteurs placés sur le textile, ils mesurent des données d'activité et physiologiques en temps réel.

Les sociétés Google et Levi's ont collaboré pour produire une veste connectée utilisant une approche différente, se basant sur l'utilisation de fils conducteurs. Cette veste permet d'interagir avec un téléphone par différents types de contacts avec la manche.

Une autre application possible des vêtements connectés est la capture de mouvement et posture humaine. Les nombreuses solutions proposées se basent généralement sur des caméras ou des capteurs inertiels. Ces solutions présentent de nombreux désavantages. Par exemple, les solutions basées sur l'analyse d'images souffrent d'un encombrement important lorsqu'elles sont précises, ou d'une grande imprécision lorsqu'elles sont purement optiques. De même, les solutions à base de capteurs inertiels sont peu précises et compliquées à calibrer.

Quelques techniques intéressantes ont été proposées en robotique comme la Tomographie par Impédance Électrique (TIE) basée sur la reconstruction du champ électrique à la surface d'un matériau conducteur. Cette technique non-invasive est déjà utilisée en imagerie médicale pour détecter les corps internes en appliquant les électrodes à la surface de la peau d'un patient et en mesurant les variations du champ électrique.

La Tomographie par Impédance Électrique (TIE) a été utilisée dans les articles de Kato et al. ("Tactile sensor without wire and sensing element in the tactile region based on eit method", IEEE Sensors, pages 792-795, 2007), et de Yao et Soleimani ("A pressure mapping imaging device based on electrical impedance tomography of conductive fabrics", Sensor Review, 32(4):310-317, 2012) pour proposer des capteurs tactiles (capteurs de pression). Les articles de Nagakubo et al. ("A deformable and deformation sensitive tactile distribution sensor", IEEE International Conference on Robotics and Biomimetics, ROBIO, pages 1301-1308, 2007), de Alirezaei et al. ("A highly stretchable tactile distribution sensorfor smooth surfaced humanoids", 7th IEEE-RAS International Conference on Humanoid Robots, pages 167-173, 2007 et "A tactile distribution sensor which enables stable measurement under high and dynamic stretch", IEEE Symposium on 3D User Interfaces (3DUI), pages 87-93, 2009), et de Tawil et al. ("Improved image reconstruction for an eit-based sensitive skin with multiple internal electrodes", IEEE Transactions on Robotics, 27(3):425-435, 2011), ont proposé des dispositifs tactiles de type « peau artificielle » pour robots. Leur approche consiste à injecter des courants et à mesurer des tensions à partir d'électrodes connectées sur les bords d'un tissu conducteur, puis à appliquer l'analyse du problème inverse pour reconstruire le changement local de la résistivité due à une pression. Enfin, les articles de Pugach et al. ("Electronic hardware design of a low cost tactile sensor device for physical human-robot interactions", IEEE XXXIII International Scientific Conference Electronics and Nanotechnology, ELNANO, pages 445-449, 2013, "Neural learning of the topographic tactile sensory information of an artificial skin through a self-organizing map", Advanced Robotics, 29(21):1393-1409, 2015, et "Touch-based admittance control of a robotic arm using neural learning of an artificial skin", In 2016 IEEE/RSJ International Conference on Intelligent Robots and Systems (IROS), pages 3374-3380, 2016) ont décrit l'utilisation de réseaux de neurones pour reconstruire la distribution de résistance au sein d'un film conducteur et localiser des points de pression. D'autres applications de la TIE, notamment à des peaux artificielles, sont décrites dans les articles de Russo Stefania et al. "Towards a practical implementation of EIT-based sensors using artificial neural networks" (2017 IEEE SENSORS, IEE, 29 octobre 2017 (2017-10-29), pages 1-3) et "Towards the Development of an EIT-based Stretchable Sensor for Multi-Touch Industrial Human-Computer Interaction Systems" (19 juin 2016 (2016-06-19), International Conference on computer analysis of images and patterns, pages 563-573) ainsi que dans la thèse de Francesco Visentin et al. "The Development of a Flexible Sensor for Continuum Soft-Bodied Robots" (31 décembre 2017 (2017-12-31)).

Toutes les applications de la TIE aux vêtements intelligents sont donc exclusivement restreintes à la mesure de pressions. Aucune application n'existe qui permette de mesurer une extension.

Cependant, aucune de ces applications ne permet de mesurer de manière fiable des activités telles que le mouvement (par exemple l'extension) d'un bras, d'une épaule ou d'un genou. Ce genre de mesure est particulièrement important dans le cadre de la prévention sanitaire, par exemple la prévention des troubles musculosquelettiques (ou TMS).

L'invention vient améliorer la situation. A cet effet, l'invention propose un textile adapté pour la détection de mouvement et/ou de déformation qui comprend un tissu conducteur électriquement et extensible selon au moins deux directions, des électrodes disposées de manière sensiblement régulière le long de la périphérie du tissu, un contrôleur agencé pour commander l'excitation des électrodes deux par deux selon un schéma tel que toutes les électrodes sont successivement excitées et pour mesurer à chaque fois la tension dans les électrodes non excitées, et un calculateur comprenant un moteur d'inférence de réseau neuronal et agencé pour recevoir les mesures de tension aux électrodes non excitées pour un cycle d'excitation, pour les fournir au moteur d'inférence de réseau neuronal et pour renvoyer une mesure caractéristique d'un mouvement ayant entraîné une déformation du textile.

En effet, en appliquant les principes de la méthode de TIE, il est possible de déterminer un mouvement de l'articulation au niveau de laquelle est placé le tissu conducteur extensible, et ainsi de réaliser un vêtement intelligent pour la capture de mouvements humains.

Dans diverses variantes, le vêtement selon l'invention peut présenter une ou plusieurs des caractéristiques suivantes :
- le contrôleur est agencé pour exciter les électrodes selon un schéma d'excitation de TIE choisi parmi le groupe comprenant un schéma de voisinage, un schéma d'opposition et un schéma transverse,
- le moteur d'inférence de réseau neuronal a fait l'objet d'un apprentissage avec des mesures caractéristiques d'un mouvement ayant entraîné une déformation du textile et des mesures de tension des électrodes non excitées obtenues selon le même schéma d'excitation que celui appliqué par le contrôleur,
- le réseau neuronal présente un perceptron,
- le textile comprend en outre deux démultiplexeurs et deux multiplexeurs commandés par le contrôleur pour mettre en œuvre le schéma d'excitation des électrodes et de mesure des tensions des électrodes non excitées.
- le textile comprend en outre une source de courant pour générer le courant utilisé par les démultiplexeurs pour l'excitation des électrodes, et
- le calculateur est agencé pour retourner une mesure articulaire.

L'invention concerne également un vêtement intelligent comprenant un tissu sensiblement non conducteur électriquement et un textile tel que décrit plus haut fixé sur le tissu, mais également un capteur de déformation comprenant un textile tel que décrit plus haut.

D'autres caractéristiques et avantages de l'invention apparaîtront mieux à la lecture de la description qui suit, tirée d'exemples donnés à titre illustratif et non limitatif, tirés des dessins sur lesquels :
- la figure 1 représente une vue schématique d'un vêtement incorporant un textile adapté pour la détection de mouvement selon l'invention,
- la figure 2 représente une vue schématique du textile adapté pour la détection de mouvement de la figure 1, et
- la figure 3 représente les résultats de la mesure angulaire grâce au textile de l'invention, et l'erreur réelle mesurée.

Les dessins et la description ci-après contiennent, pour l'essentiel, des éléments de caractère certain. Ils pourront donc non seulement servir à mieux faire comprendre la présente invention, mais aussi contribuer à sa définition, le cas échéant.

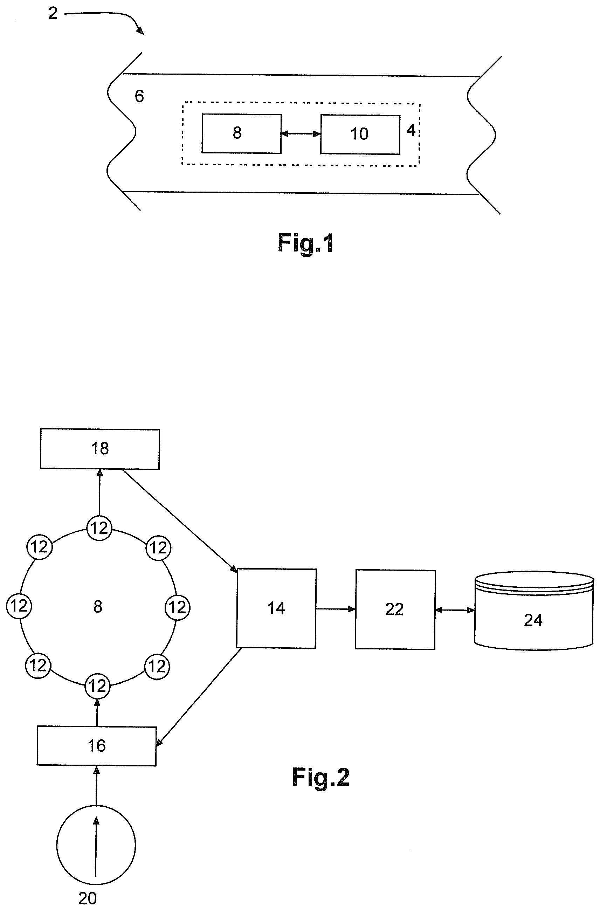

La figure 1 représente une vue schématique d'un vêtement intelligent 2 incorporant un textile 4 adapté pour la détection de mouvement. Le vêtement 2 comprend un tissu 6 non conducteur électriquement ou isolant sur lequel est fixé le textile 4. La fixation du textile 4 sur le tissu 6 peut être réalisé de toute manière appropriée, qu'il s'agisse d'une couture, d'un collage, d'une fusion partielle ou d'un entre tissage sur à la périphérie du textile 4.

Le textile 4 comprend un tissu 8 et un module de mesure d'extension 10. Le tissu 8 est dans l'exemple décrit ici du type EeonTex (marque déposée, tissu vendu par la société Eeonyx sous la référence EeonTex LTT-SLPA), et est un tissu conducteur extensible de manière bi-directionnelle composé à 72 % de nylon, et à 28% de spandex. Il présente une masse par unité de surface d'environ 162,72 g/m², une épaisseur d'environ 0,38mm, un taux de récupération d'élongation de 85% et une résistance surfacique pouvant être réglée par traitement de surface entre 10000 ohms/pouce² et 10 000 000 ohms/pouce². En variante, tout autre tissu conducteur extensible présentant des capacités à s'étendre dans plusieurs directions (c'est-à-dire ne présentant pas une direction d'élongation préférentielle telle que le tissu ne s'étend sensiblement que dans cette direction préférentielle) pourra être utilisé.

Le tissu 8 est connecté à une pluralité d'électrodes qui sont reliées au module de mesure d'extension 10. La figure 2 permet de mieux représenter le textile 4 et la relation entre le tissu 8 et le module de mesure d'extension 10.

Comme on peut le voir sur la figure 2, le tissu 8 présente une forme sensiblement circulaire et reçoit dans l'exemple décrit ici huit électrodes 12 réparties de manière sensiblement homogène à la périphérie du tissu 8. La forme sensiblement circulaire est particulièrement adaptée pour réaliser des mesures sur un coude ou un genou. En variante, le textile 4 pourrait comprendre moins d'électrodes, par exemple 4, ou plus, par exemple 16 ou plus.

Le module de mesure d'extension 10 comprend dans l'exemple décrit ici un contrôleur 14, deux démultiplexeurs 16, deux démultiplexeurs 18, une source 20, un calculateur 22 et une mémoire 24.

Dans l'exemple décrit ici, le contrôleur 14 est un microcontrôleur du type ARM 32 bits (par exemple Atmel SAM3X8E ARM utilisant un processeur Cortex-M3 RISC). Le contrôleur 14 comprend un convertisseur analogique-numérique sur 12 bits permettant d'échantillonner les signaux qu'il reçoit. Le contrôleur 14 a pour rôle de commander les démultiplexeurs 16 et les multiplexeurs 18 afin d'une part d'exciter séquentiellement les électrodes 12 par paire, en déplaçant la masse à chaque fois, et d'autre part de mesurer la chute de tension aux bornes des autres électrodes 12.

Les démultiplexeurs 16 et les multiplexeurs 18 sont dans l'exemple décrit ici du type MAX306CPI+ de Maxim Integrated. Ils ont pour fonction respectivement le démultiplexage des signaux d'excitation émis par le contrôleur 14, et le multiplexage des signaux de mesure au niveau des électrodes 12 comme décrit plus haut. D'un point de vue fonctionnel, on peut considérer que les démultiplexeurs 16 et les multiplexeurs 18 sont couplés au contrôleur 14, dans la mesure où ils remplissent ensemble la fonction d'excitation et de mesure.

Le contrôleur 14 est agencé pour réaliser l'excitation selon un schéma d'excitation de TIE afin d'induire dans le tissu 8 un changement de résistance au niveau des électrodes 12 qui est caractéristique de l'extension de celui-ci. La source de courant 20 fournit aux démultiplexeurs 16 le courant qui est démultiplié pour les courants d'excitation. La source de courant 20 peut être continue, auquel cas la tension de mesure sera mesurée simultanément dans les électrodes 12, ou alternative, auquel cas seront mesurées l'amplitude et le décalage de la tension par rapport au courant alternatif. En variante, la source de courant 20 pourrait être omise. Toujours en variante, les démultiplexeurs et les multiplexeurs pourraient également être omis en utilisant un contrôleur connecté directement ou par indirectement aux électrodes.

Par schéma d'excitation de TIE on entend un schéma choisi parmi :
- un schéma de voisinage selon lequel le courant d'excitation est introduit dans des électrodes voisines, et la chute tension est mesurée successivement dans les autres électrodes, chaque paire d'électrode étant successivement utilisée pour réaliser une excitation,
- un schéma d'opposition selon lequel le courant d'excitation est introduit dans des électrodes diamétralement opposées, et la tension est mesurée successivement dans les autres électrodes, chaque paire d'électrode étant successivement utilisée pour réaliser une excitation, et
- un schéma transverse selon lequel le courant d'excitation est introduit dans des électrodes opposées par rapport à un axe fixé, et la tension est mesurée successivement dans les autres électrodes, chaque paire d'électrode étant successivement utilisée pour réaliser une excitation.

Les travaux de la Demanderesse ont révélé que le schéma dit de voisinage est celui offrant les meilleurs résultats. Les travaux de la Demanderesse suggèrent que cela provient du fait que ce schéma offre un bon compromis entre la sensibilité et la sélectivité.

L'utilisation d'un microcontrôleur du type décrit plus haut permet de garder des coûts de production modestes, ce qui permet d'industrialiser la production du textile 4. En variante, le contrôleur 14 pourrait être remplacé par un autre microcontrôleur ou par un code exécuté par un processeur. Par processeur, il doit être compris tout processeur adapté aux opérations du contrôleur 14. Un tel processeur peut être réalisé de toute manière connue, sous la forme d'un microprocesseur pour ordinateur personnel, d'une puce dédiée de type FPGA ou SoC (« system on chip » en anglais), d'une ressource de calcul sur une grille, d'un microcontrôleur, ou de toute autre forme propre à fournir la puissance de calcul nécessaire à la réalisation décrite plus bas. Un ou plusieurs de ces éléments peuvent également être réalisés sous la forme de circuits électroniques spécialisés tel un ASIC. Une combinaison de processeur et de circuits électroniques peut également être envisagée.

Dans l'exemple décrit ici, le contrôleur 14, les démultiplexeurs 16, les multiplexeurs 18 et la source de courant 20 permettent d'acquérir des données à une fréquence de 45Hz.

Selon la méthode TIE, le courant qui passe à travers le tissu crée une distribution volumique du potentiel électrique. Le potentiel diminue le long de la ligne de courant en fonction de la distance par rapport aux électrodes actives entre lesquelles le courant est injecté. La chute de tension par unité de longueur (intensité du champ électrique) est proportionnelle à l'intensité du courant et la résistance du milieu en conformité avec la loi d'Ohm. En mesurant la chute de tension et en connaissant la valeur du courant, la valeur de résistance peut alors être calculée. Un algorithme tomographique de reconstruction permet d'utiliser les tensions mesurées seulement à la surface de tissu pour calculer la distribution spatiale de la résistivité à l'intérieur de ce dernier.

Néanmoins, le modèle qui décrit la corrélation entre la posture/le mouvement et la déformation du tissu 8 est difficile à obtenir analytiquement. Pour cette raison, la Demanderesse a eu l'idée d'utiliser le calculateur 22 en combinaison à la mémoire 24.

Le calculateur 22 a pour fonction d'appliquer le modèle d'inférence d'un réseau neuronal aux mesures de chute de tension aux électrodes 12. Ainsi, en entraînant un réseau neuronal sur des milliers de mouvements et de mesures de chute de tension correspondantes, il devient possible de s'affranchir de la détermination du modèle. Plus précisément, la Demanderesse a découvert qu'un réseau neuronal de type LMS (pour « Least Mean Squares » en anglais, par exemple un réseau neuronal avec un perceptron sans filtrage softmax en sortie), utilisé dans le paradigme d'apprentissage conditionnel, permet de prédire l'angle articulaire avec une précision de plus ou moins 5 degrés.

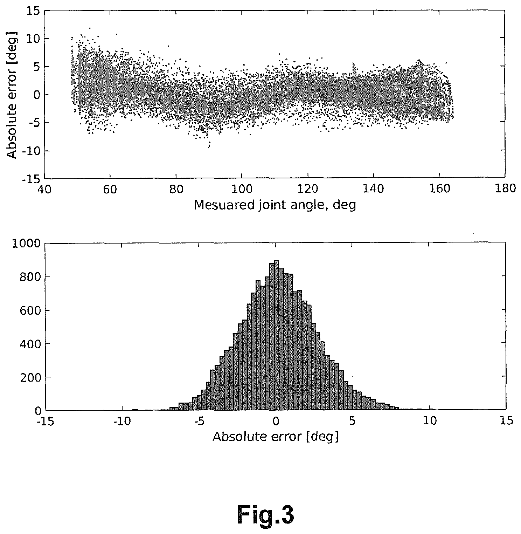

La figure 3 permet de voir les résultats de la mesure angulaire grâce au textile de l'invention, et l'erreur réelle mesurée.

Par paradigme d'apprentissage conditionnel, on entend ici une méthode d'optimisation qui consiste en la modification des poids synaptiques du réseau neuronal jusqu'à trouver l'erreur quadratique moyenne minimale entre l'entrée et la sortie désirée. Ainsi, en connaissant l'angle articulaire d'entrée, l'apprentissage du réseau neuronal lui permet d'apprendre à prédire une sortie désirée dérivée d'un stimulus inconditionnel et d'associer cette sortie à un stimulus conditionnel. Cette architecture est équivalente à un conditionnement pavlovien, qui associe la prédiction d'un angle articulaire à la distribution de la résistance dans le tissu 8. En réalisant un apprentissage selon un schéma d'excitation de TIE, l'apprentissage permet donc de reproduire fidèlement l'approche de cette méthode. Par ailleurs, un autre type de réseau neuronal peut être appliqué afin d'assosier l'extension de tissu avec l'angle articulaire, par exemple un réseau neuronal supervisé ou un réseau neuronal convolutif.

L'utilisation d'un réseau neuronal présente de plus l'avantage de permettre d'utiliser un calculateur 22 plus simple, et donc moins coûteux, puisque l'application d'un moteur d'inférence d'un réseau neuronal à un perceptron est bien moins lourd en calcul que la résolution du problème inverse de la méthode TIE classique. Dans l'exemple décrit ici, le calculateur 22 est un ordinateur du type Raspberry ou tout autre ordinateur léger à coût modeste et propre à mettre en œuvre le moteur d'inférence du réseau neuronal à un perceptron avec les mesures de tensions issues du contrôleur 14. Dans l'exemple décrit ici, le calculateur 22 est décrit de façon principielle, et la mémoire 24 stocke le moteur d'inférence du réseau neuronal à un perceptron, les mesures de tensions issues du contrôleur 14, et les mesures d'angle résultantes. La mémoire 24 peut être tout type de stockage de données propre à recevoir des données numériques : disque dur, disque dur à mémoire flash (SSD en anglais), mémoire flash sous toute forme, mémoire vive, disque magnétique, stockage distribué localement ou dans le cloud, etc. De manière préférée, elle est réalisée par la mémoire du calculateur 22.

Ainsi, le textile selon l'invention :
- Est peu intrusif et peu encombrant,
- Est peu coûteux par rapport aux systèmes qui utilisent des caméras,
- Est peu limité par une zone de visibilité de capture (ce qui est souvent le cas des capteurs externes), et indépendant des occlusions (à l'inverse des systèmes qui utilisent des caméras),
- Permet de réaliser la mesure longitudinale, contrairement aux systèmes avec les capteurs inertiels, qui sont soumis aux dérives,
- Est dépourvu de tout capteur autre que les électrodes, et
- Compatible avec une utilisation dans le milieu industriel.

De plus, grâce la simplification de la méthode TIE par l'utilisation de réseaux neuronaux, il est simple à mettre en œuvre et peu gourmand en ressources de calcul. L'utilisation des réseaux neuronaux garantit également son évolutivité et la possibilité de créer de nombreux profils adaptés à des mesures distinctes par l'entraînement de plusieurs moteurs d'inférence spécialisés pour des conditions spécifiques.

Le textile intelligent selon l'invention peut de manière plus générale être utilisé dans un capteur de déformation qui peut être utilisé pour quantifier la variation de la forme d'un objet souple ou d'une chaîne articulée multicorps. Par exemple, un tel capteur pourrait être utilisé dans un robot série, un robot souple (« *soft robotics* » en anglais), pour la mesure de déformation de fauteuils (application automobile ou aéronautique), etc.

## Revendications

1. Textile adapté pour la détection d'un mouvement et/ou d'une déformation d'un corps sur lequel est agencé ledit textile, **caractérisé en ce qu'**il comprend un tissu (8) conducteur électriquement et extensible selon au moins deux directions, des électrodes (12) disposées de manière sensiblement régulière le long de la périphérie du tissu (8), un contrôleur (14) agencé pour commander l'excitation des électrodes (12) deux par deux selon un schéma tel que toutes les électrodes (12) sont successivement excitées et pour mesurer à chaque fois la tension dans les électrodes (12) non excitées, et un calculateur (22) comprenant un moteur d'inférence de réseau neuronal et agencé pour recevoir les mesures de tension aux électrodes (12) non excitées pour un cycle d'excitation, pour les fournir au moteur d'inférence de réseau neuronal et pour renvoyer au moins une valeur, déterminée par le moteur d'inférence de réseau neuronal, représentative du mouvement et/ou de la déformation du corps ayant entraîné une déformation du textile.

2. Textile selon la revendication 1, dans lequel le contrôleur (12) est agencé pour exciter les électrodes (12) selon un schéma d'excitation de TIE choisi parmi le groupe comprenant un schéma de voisinage, un schéma d'opposition et un schéma transverse.

3. Textile selon la revendication 1 ou 2, dans lequel le moteur d'inférence de réseau neuronal a fait l'objet d'un apprentissage avec des mesures caractéristiques d'un mouvement ayant entraîné une déformation du textile et des mesures de tension des électrodes (12) non excitées obtenues selon le même schéma d'excitation que celui appliqué par le contrôleur (14).

4. Textile selon la revendication 3, dans lequel le réseau neuronal présente un perceptron.

5. Textile selon l'une des revendications précédentes, comprenant en outre deux démultiplexeurs (16) et deux multiplexeurs (18) commandés par le contrôleur (14) pour mettre en œuvre le schéma d'excitation des électrodes (12) et de mesure des tensions des électrodes (12) non excitées.

6. Textile selon la revendication 5, comprenant en outre une source de courant (20) pour générer le courant utilisé par les démultiplexeurs (16) pour l'excitation des électrodes (12).

7. Textile selon l'une des revendications précédentes, dans lequel le corps sur lequel le textile est agencé comprend une articulation, et l'au moins une valeur retournée par le calculateur (22) comprend une valeur d'un angle articulaire représentative du mouvement articulaire de l'articulation.

8. Vêtement intelligent, comprenant un tissu (6) sensiblement non conducteur électriquement et un textile (4) selon l'une des revendications précédentes fixé sur le tissu (6).

9. Capteur de déformation, comprenant un textile (4) selon l'une des revendications précédentes.

## Patentansprüche

1. Textil, das zur Detektion einer Bewegung und/oder einer Verformung eines Körpers, auf dem das Textil angeordnet ist, geeignet ist, **dadurch gekennzeichnet, dass** es ein elektrisch leitendes und in mindestens zwei Richtungen dehnbares Gewebe (8), im Wesentlich regelmäßig entlang des Randes des Gewebes (8) angeordnete Elektroden (12), eine Steuerung (14), die dazu ausgestaltet ist, die paarweise Anregung der Elektroden (12) gemäß einem derartigen Schema zu steuern, dass alle Elektroden (12) nacheinander angeregt werden, und jedes Mal die Spannung in den nicht angeregten Elektroden (12) zu messen, und einen Rechner (22), der eine Inferenzmaschine eines neuronalen Netzes umfasst und dazu ausgestaltet ist, die Spannungsmessungen an den bei einem Anregungszyklus nicht angeregten Elektroden (12) zu empfangen, um sie an die Inferenzmaschine des neuronalen Netzes zu übermitteln, und mindestens einen von der Inferenzmaschine des neuronalen Netzes ermittelten Wert zurückzusenden, der für die Bewegung und/oder die Verformung des Körpers, die zu einer Verformung des Textils geführt hat, repräsentativ ist, umfasst.

2. Textil nach Anspruch 1, wobei die Steuerung (12) dazu ausgestaltet ist, die Elektroden (12) gemäß einem EIT-Anregungsschema anzuregen, das aus der Gruppe gewählt ist, die ein Benachbart-Schema, ein Entgegengesetzt-Schema und ein transversales Schema umfasst.

3. Textil nach Anspruch 1 oder 2, wobei die Inferenzmaschine des neuronalen Netzes Gegenstand eines Trainings mit charakteristischen Messungen einer Bewegung, die zu einer Verformung des Textils geführt hat, und Spannungsmessungen der nicht angeregten Elektroden (12), die nach dem gleichen Anregungsschema wie dem von der Steuerung (14) angewandten erhalten wurden, gewesen ist.

4. Textil nach Anspruch 3, wobei das neuronale Netz ein Perzeptron aufweist.

5. Textil nach einem der vorhergehenden Ansprüche, umfassend ferner zwei Demultiplexer (16) und zwei Multiplexer (18), die von der Steuerung (14) gesteuert werden, um das Schema zur Anregung der Elektroden (12) und zur Messung der Spannungen der nicht angeregten Elektroden (12) zu implementieren.

6. Textil nach Anspruch 5, umfassend ferner eine Stromquelle (20), um den von den Demultiplexern (16) zum Anregen der Elektroden (12) verwendeten Strom zu erzeugen.

7. Textil nach einem der vorhergehenden Ansprüche, wobei der Körper, auf dem das Textil angeordnet ist, ein Gelenk umfasst und der mindestens eine von dem Rechner (22) zurückgeschickte Wert einen Wert eines Gelenkwinkels umfasst, der für die Gelenkbewegung des Gelenks repräsentativ ist.

8. Intelligentes Kleidungsstück, umfassend ein im Wesentlichen nicht elektrisch leitendes Gewebe (6) und ein Textil (4) nach einem der vorhergehenden Ansprüche, das an dem Gewebe (6) fixiert ist.

9. Verformungssensor, der ein Textil (4) nach einem der vorhergehenden Ansprüche umfasst.

## Claims

1. Textile suitable for detecting a movement and/or a deformation of a body part on which said textile is arranged, **characterized in that** it comprises an electrically conductive fabric (8) which can be extended in at least two directions, electrodes (12) arranged substantially regularly along the periphery of the fabric (8), a controller (14) designed to control the excitation of the electrodes (12), two by two, according to a pattern such that all the electrodes (12) are successively excited, and to measure each time the voltage in the non-excited electrodes (12), and a calculator (22) comprising a neural network inference engine and designed to receive the voltage measurements taken at the non-excited electrodes (12) in a given excitation cycle, in order to supply them to the neural network inference engine and to return at least one value, determined by the neural network inference engine, representative of the movement and/or the deformation of the body part having caused the movement and/or deformation of the textile.

2. Textile according to claim 1, wherein the controller (12) is designed to excite the electrodes (12) according to an EIT excitation pattern chosen from the group comprising an adjacent pattern, an opposite pattern and a transverse pattern.

3. Textile according to claim 1 or 2, wherein the neural network inference engine undergoes learning with measurements characteristic of a movement having caused a deformation of the textile and voltage measurements taken at the non-excited electrodes (12) obtained according to the same excitation pattern as that applied by the controller (14).

4. Textile according to claim 3, wherein the neural network has one perceptron.

5. Textile according any one of the preceding claims, further comprising two demultiplexers (16) and two multiplexers (18) controlled by the controller (14) in order to implement the excitation pattern of the electrodes (12) and voltage measurements taken at the non-excited electrodes (12).

6. Textile according to claim 5, further comprising a current source (20) for generating the current used by the demultiplexers (16) in order to excite the electrodes (12).

7. Textile according to any one of the preceding claims, wherein the body part on which the textile is arranged comprises a joint, and the at least one value returned by the calculator (22) comprises a value of a joint angle representative of the joint movement of the joint..

8. Smart garment, comprising a substantially electrically non-conductive fabric (6) and a textile (4) according to any one of the preceding claims fixed on the fabric (6).

9. Deformation sensor, comprising a textile (4) according to any one of the preceding claims.
